Europäisches Patentamt

European Patent Office

Office européen des brevets

(11) Publication number: **0 183 462 B1**

# EUROPEAN PATENT SPECIFICATION

(45) Date of publication of patent specification: **28.08.91**  (51) Int. Cl.<sup>5</sup>: **G06F 3/16, A61H 31/00**

(21) Application number: **85308382.2**

(22) Date of filing: **18.11.85**

(54) **Cardiopulmonary resuscitation prompting apparatus and method.**

(30) Priority: **21.11.84 US 673838**

(43) Date of publication of application:
**04.06.86 Bulletin 86/23**

(45) Publication of the grant of the patent:
**28.08.91 Bulletin 91/35**

(84) Designated Contracting States:
**AT BE CH DE FR GB IT LI LU NL SE**

(56) References cited:
**EP-A- 0 162 616      EP-A- 0 186 713
US-A- 4 016 540      US-A- 4 193 064
US-A- 4 360 345      US-A- 4 420 813**

(73) Proprietor: **Hutchins, DonaldC.**
**1047 Longmeadow Street**
**Longmeadow Massachusetts 01106(US)**

(72) Inventor: **Hutchins, DonaldC.**
**1047 Longmeadow Street**
**Longmeadow Massachusetts 01106(US)**

(74) Representative: **Brunner, Michael John et al**
**GILL JENNINGS & EVERY 53-64 Chancery**
**Lane**
**London WC2A 1HN(GB)**

## Description

This invention relates to cardiopulmonary resuscitation (CPR) and, more particularly, concerns apparatus and techniques for prompting one or more rescuers to follow properly rescue procedures, such as those prescribed by the American Heart Association in conjunction with the American Red Cross, with apparatus which is relatively easy to operate by relatively unskilled personnel to provide major assistance during emergency conditions requiring CPR efforts by nonmedical personnel.

These procedures are based on information provided by the Division of Medical Science, National Academy of Sciences, U.S. National Research Council.

Cardiopulmonary resuscitation is a combination of artificial respiration and artificial circulation which should be started as an emergency procedure, when cardiac arrest occurs, by those properly trained to do so.

To understand the principles and techniques of cardiopulmonary resuscitation, it is important to know some of the concepts involves in this procedure.

The following points are important to remember:

1. Air that enters the lungs contains about 21 percent oxygen and only a trace of carbon dioxide. Air that is exhaled from the lungs contains about 16 percent oxygen and 4 percent carbon dioxide.
2. The right side of the heart pumps blood to the lungs where the blood picks up oxygen and releases carbon dioxide.
3. The oxygenated blood then returns to the left side of the heart, from where it is pumped to the tissues of the body.
4. In the body tissues, the blood releases oxygen and taken up carbon dioxide, after which it flows back to the right side of the heart.
5. All body tissues require oxygen, but the brain requires more than any other tissue. It is generally estimated that if the brain is totally deprived of oxygenated blood for a period of four to six minutes, it will probably suffer irreversible damage.
6. The condition that exists when both breathing and circulation stop is called clinical death.
7. The condition that exists when the brain has been deprived of oxygenated blood for a period of six minutes or more and irreversible damage has probably occurred is called biological death.

Both ventilation and circulation are required to maintain life.

When breathing stops, the circulation and pulse may continue for some time, a condition known as respiratory arrest. In this case, only artificial respiration is required, since the heat action continues to circulate blood to the brain and the rest of the body. Common causes of respiratory arrest are drowning, electric shock, suffocation, strangulation, accidents and drug overdosage.

When circulation stops, the pulse disappears and breathing stops at the same time or soon thereafter. This condition is known as cardiac arrest. When cardiac arrest occurs, both artificial respiration and artificial circulation are required to oxygenate the blood and circulate it to the brain. Common causes of cardiac arrest are heart attack; electric shock; haemorrhage; and, as a final phase of drowning, suffocation and other forms of respiratory arrest.

There are some situations which require special actions for the artificial resuscitation; one of these is choking. It is during eating that obstruction of the airway by foreign bodies most often occurs. In adults, meat is the most common object that causes obstruction; but, in children, and in some adults, a variety of other foods and foreign bodies may lead to a blockage of the airway.

A number of years ago, the American Red Cross (ARC) and the American Heart Association (AHA) developed a Statement of Cooperation between the two organisations. Under this Agreement, AHA assumed the role of research and ARC developed instruction programs in CPR to train nonprofessional rescuers. Under these programs, millions have been trained and thousands of lives have been saved.

To be certified as a CPR rescuer, a person is taught a series of physical procedures that cause artificial breathing and circulation. These procedures, such as chest thrusts, are called for in prescribed sequences and with specific timing. The sequence and timing are important because they are directly related to a standard pulse beat needed to prevent bilogical death.

United States authorities have agreed upon a method of CPR. For training purposes, most instructors furnish the Red Cross CPR Module, the standard for CPR training in the United States.

CPR procedures differ depending upon the condition and age of the victim and also the number of trained CPR rescuers present. For example, in a one-rescuer situation, the Module asks for a rate of 80 compressions per minute for a child. With two-rescuer CPR, 60 compressions per minute are required. US-A-4193064 discloses apparatus of the general type that may be used in such procedures.

These cycles are interrupted at specified intervals when diagnoses of vital signs are required and when a different breathing pattern is needed. While all these directions burden the rescuer's mind, he must remember such important steps as: "Keep the airway passage open", "Check for pulse", "Seal off the nose", "Tilt the head back", and other steps.

The single biggest challenge remaining in CPR training is to find a solution to the problem of skill and knowledge decay. The Red Cross has promoted recertification classes each year after the initial course, and this has been a partial solution to this problem. However, in reality, only a small percentage attend these recertification classes. As a result, millions of people trained in CPR are rusty when it comes to instant recall of the routines, timing and sequence of actions.

There are also those who have the knowledge but who panic or go blank under the stress of an emergency. It is one thing to have the knowledge, and something quite different to recall and follow correct procedures when a loved one is unconscious, lifeless, or choking.

Various methods of prompting have been promoted, yet all established solutions have flaws which limit their success. One idea is a simple wallet-size card that those trained in CPR can carry and read. Unfortunately, the small size of the cards limits the number of instructions they can carry, and the rescuer must divert his attention from the victim to read the prompts.

Audio instruction tapes, while useful in the classroom, are of little or no help in emergencies. Tapes are recorded sequentially, and tape recorders lack the search techniques needed to find and produce the proper instruction sequence demanded for a particular situation.

Telephone "hot lines" have been used experimentally in some cities. Generally when money from the funding grants has been used up, the CPR "hot lines" have closed down.

Emergency medical technicians have made good use of two-way radio and cable networks. When patched into hospital facilities, both instructions and diagnostic information can be transmitted. Unfortunately, a victim of cardiac arrest has only 4 minutes before brain damage is probable. In most of these cases, professional help arrives too late. The need is for immediate attention from a CPR-trained bystander.

From EP-A-0162616 it is known to provide a portable device which will give audible prompts to those previously trained in CPR, capable of moving to and with the victim.

EP-A-0162616 (now abandoned), which is relevant only in regard to the question of novelty, having been published after the priority date of this application, but having earlier filing and priority dates respectively and therefore being relevant by virtue of EPC Article 54(3) only, shows cardiopulmonary resuscitation prompting apparatus of the general type (see US-A-4193064) which includes keying means for entering information signals representative at least of characteristics of a victim relevant to proper performance of cardiopulmonary resuscitation techniques; and means responsive to the keyed information signals for providing output signals representative of proper steps to be taken in resuscitating a victim; and electroacoustical transducing means responsive to the output signals for providing audible intelligible speech sound signals representative of the proper resuscitation steps to be taken.

The present invention relates to such apparatus which further includes keying means for identifying whether the victim is conscious or unconscious.

Being able to indicate consciousness or unconsciousness enables the rescuer to change the CPR prompts quickly if the victim's condition changes.

An embodiment of the invention will now be described, by way of example, with reference to the accompanying drawings, in which:

Figure 1 is a block diagram illustrating the logical arrangement of apparatus according to the invention;

Figure 2 is a combined block schematic-circuit diagram of an embodiment of the invention;

Figure 3 is a block diagram illustrating the logical arrangement of an embodiment of the invention;

Figure 4 is a block diagram illustrating the logical arrangement of voice synthesizing elements;

Figure 5 is a front view of a preferred form of cabinet for housing the apparatus;

Figure 6 is a logic diagram illustrating the steps in a program for putting the invention into practice; and

Figure 7 illustrates a logical arrangement for carrying out steps in the program.

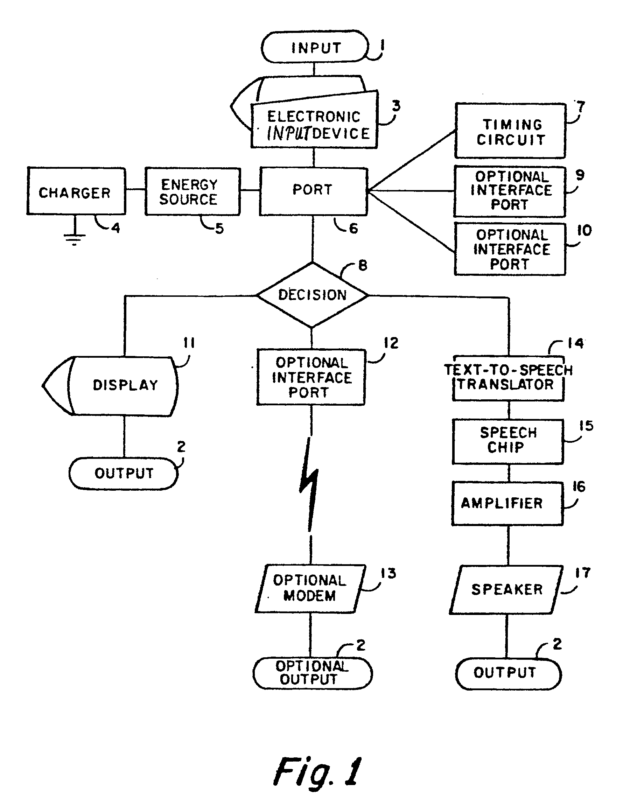

Referring to Figure 1 of the drawings, a CPR prompting apparatus comprises an electronic input device 3, which is preferably a keypad or key button for manual startup, test circuits, and program selection. A power source 5, such as batteries or a light-sensitive energizer, may provide electric power to allow portability. If the source 5 comprises batteries, a plug-type charger 4 operating from an a.c. mains supply may be provided to charge them to ensure that they are fully charged when needed.

A central processing unit 6 comprises a solid-state device programmed with the logic necessary to prompt the CPR rescuer. This programmable unit may comprise an electronic chip or series of chips that provide repetition counters, conditional responses, buffer storage, and output formats required. A timing circuit 7 provides clock pulses for timing the CPR procedures.

Some output signals are used to display words or characters on a display device 11, typically comprising DRO's or liquid crystal display (LCD) devices. Other output signals energise voice synthesizer circuitry comprising a text-to-speech translator 14, a speech chip 15, an amplifier 16 and an audio output speaker 17. There are many methods of speech synthesis such as phoneme, LPC (Linear-Predictive Coding), DPCM (Differential Pulse-Code Modulation), and ADPCM (Adaptive Differential Pulse-Code Modulation). While Figure 1 shows an analysis-synthesis technique, any other method furnishing understandable speech is suitable. For the preferred embodiment of the invention, study has shown that when an analysis-synthesis system is fabricated on a silicon chip, it offers long speech times at low cost. Using this PARCOR system, speech signal waveforms are analyzed and specific parameters are encoded in the forms of digital data, and decoded from the data for synthesis. This system has the advantage of requiring far less data memory because the data are compressed by analysis and encoding. Voice output can alternatively be provided in languages other than English, to allow international use of the invention.

Peripheral devices connected to interface ports 9 and 10 may include means for diagnosis of the victim's condition or for other desirable functions. An analog-type blood pressure and pulse rate reader may feed signals representative of blood pressure and heart rate into the CPU logic through the interface ports 9 and 10. An interface port 12 may provide output signals for diagnostic display and communication, through a modem 13, to medical professionals involved in a community emergency medical services (EMS) System.

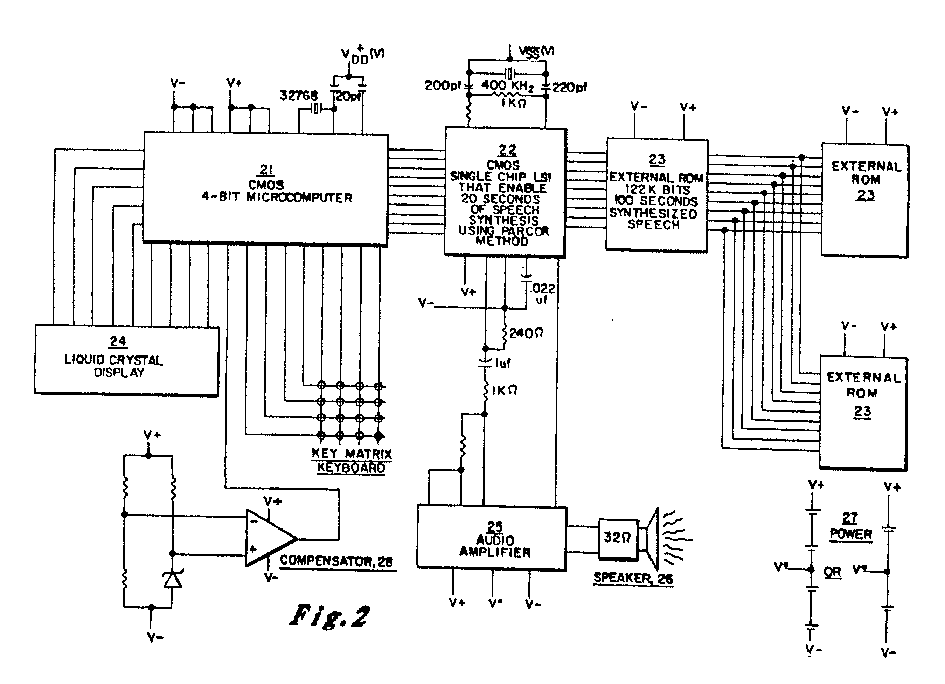

Referring to Figure 2, the CPR apparatus includes a CMOS 4-bit microcomputer 21, such as a Sanyo Electric Co. Ltd. LC 5800 CMOS LSI device with LCD driver, which operates at a low voltage and a very small current and which includes an LCD driver, a 4-bit parallel processing arithmetic logic unit, a plurality of LCD segment outputs, input/output ports, a prescaler and a 32.768kHz crystal oscillator. The microcomputer 21 provides signals to a LSI CMOS signal chip speech synthesizer 22 which can provide up to 20 seconds of synthesized speech using the known PARCOR method. The synthesizer 22 delivers these segments to an external ROM 23 which is capable of storing 128 K-bits of information to store 100 seconds of buffered synthesized speech signals. An LCD display 24 displays graphically a specific key that has been pressed by an operator and also displays messages to a rescuer in alphanumeric form.

An LSI CMOS signal chip audio amplifier 25 amplifies the synthesized speed signals provided by the synthesizer 22 and energizes a loudspeaker 26 with intelligible sounds to be used by the rescuer, which sounds may represent both speech instructions and beats for ensuring proper timing of victim-assistance manoeuvres.

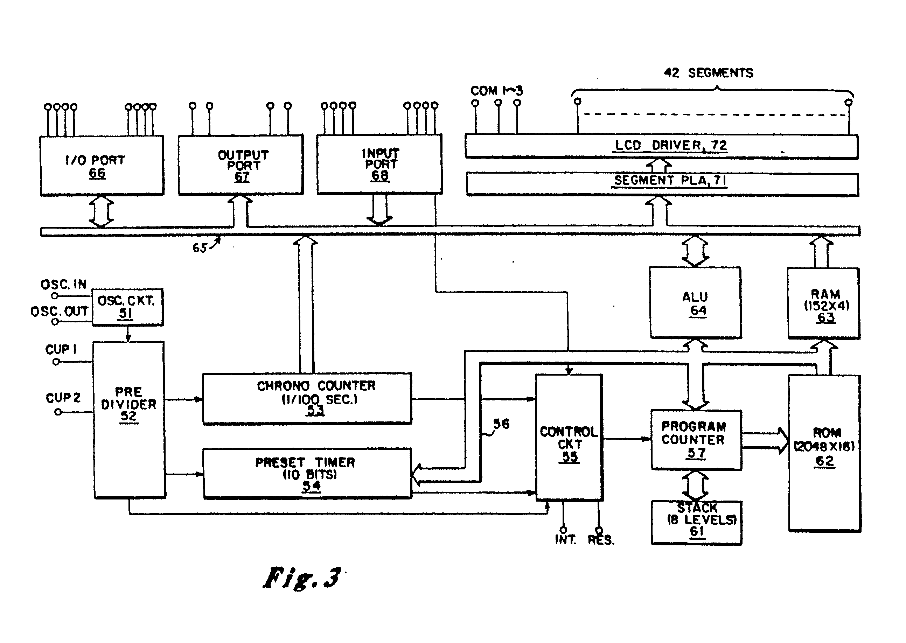

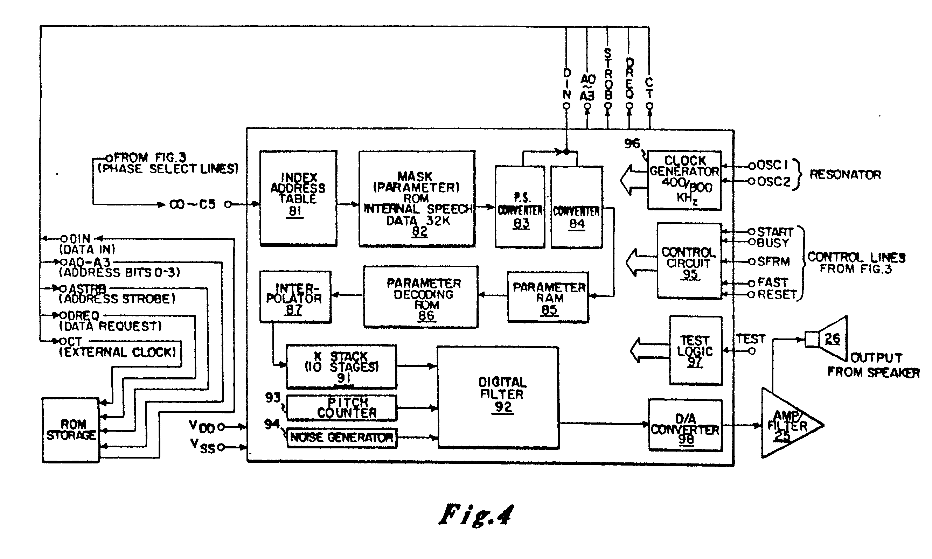

Referring to Figure 3, there is shown a block diagram illustrating the logical arrangement of the CMOS 4-bit microcomputer 21. This unit, which is a commercially available LSI device, typically comprises an oscillator circuit 51 which energizes a predivider 52. The predivider 52 energizes a chrono counter 53 with a pulse every 1/100 second and provides a preset timer 54 with clock pulses to produce a carry pulse when reset to zero to control a control circuit 55. The control circuit 55 also receives timing signals from the chrono counter 53 and appropriate signals from a bus 56. The control circuit 55 also energizes a program counter 57 which exchanges data with the bus 56, an 8-level memory stack 61 and a read only memory (ROM) 62.

The ROM 62, a random access memory (RAM) 63 and an arithmetic logic unit 64 also communicate with the bus 56. The arithmetic logic unit 64 and the RAM 63 also communicate with a bus 65 which also links an input/output (I/O) port 66, an output port 67, an input port 68 and a segment programmable logic array 71 which energizes an LCD driver 72.

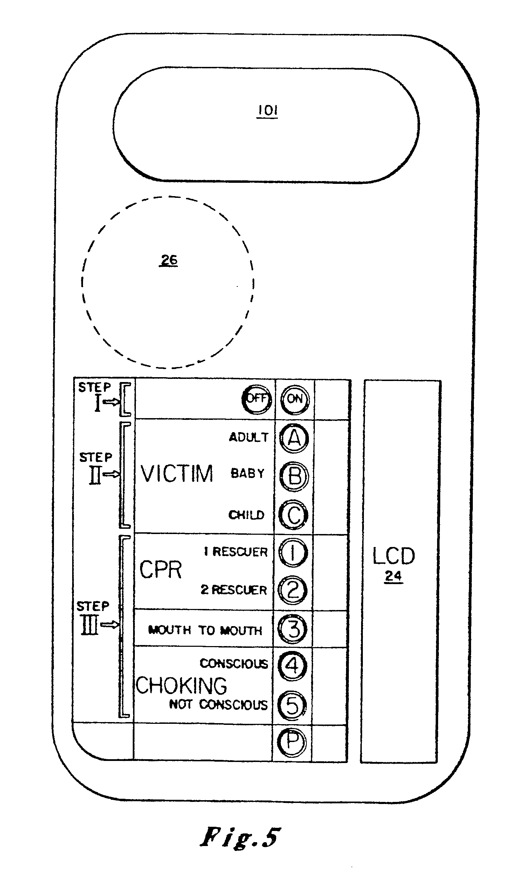

Referring to Figure 4, there is shown a block diagram illustrating the logical arrangement of the CMOS LSI signal chip speech synthesizer 22, which typically may be a Sanyo Electric Co. Ltd. type LC8100 integrated circuit, in association with the audio amplifier 25 and the loudspeaker 26. This chip typically comprises an index address table ROM 81 for specifying the indexed address for words to be generated with the CO and C5 code and a masked-parameter ROM 82 to store speech parameters. The ROM 82 feeds a parallel-serial converter 83 which drives a converter 84, which may also receive a signal on a data input (DIN) to drive a parameter RAM 85, to store parameters or one frame, which feeds a signal to a parameter decoding ROM 86. The ROM 86, which is used to decode parameters non-linearly, energizes an interpolator 87 to feed signals to a K stack 91, which is typically of 10 stages. The K stack 91 drives a digital filter 92 which also receives signals from a pitch counter 93 and a noise generator 94, which act as the voiced and unvoiced sound sources, respectively, to provide digital representations of the selected words. Those digital representations are converted by a digital-to-analog converter 98 to speech signals. The speech signals are amplified by the power amplifier 25 and are reproduced by the loudspeaker 26. The chip 22 also includes a test logic circuit 97, a control circuit 95 and a 400/800kHz clock generator 96 for providing clock pulses.

Referring to Figure 5, there is shown a preferred form of a front panel of a case of the apparatus, illustrating the preferred form of keyboard. The case is formed with an aperture 101 which defines thereabove a handle to permit convenient carrying. The keyboard includes keys designated off, on; A, B, C; 1-5; and P. The LCD display 24 displays appropriate visual information as indicated above, and the loudspeaker 26 provides appropriate aural information.

Also marked on the case are various steps to be taken in setting up the CPR procedure. Step I involves pressing the ON button. Step II involves pressing one of the buttons A, B or C to identify whether the victim to be treated is an adult, baby or child, respectively. Step III involves pressing one of buttons 1 or 2 to indicate, respectively, whether one or two rescuers are avilable for giving the treatment, pressing button 3 to indicate that mouth-to-mouth artifical respiration will be given, and pressing one of buttons 4 or 5 to indicate whether a choking victim is conscious or not conscious. Pressing the P or pause key causes the apparatus to pause in providing prompts until the rescuer is ready.

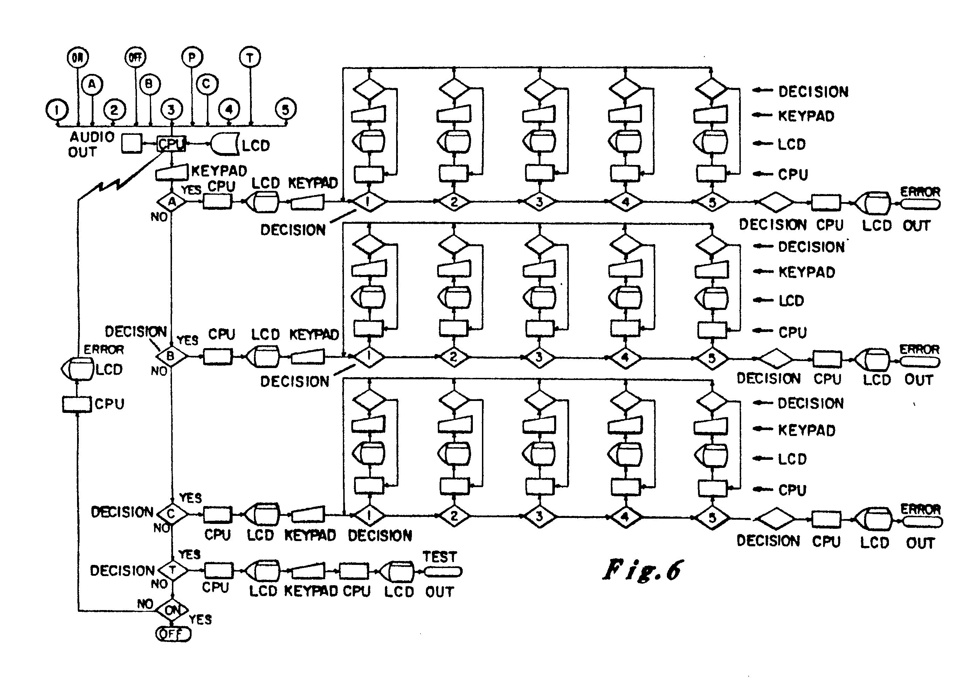

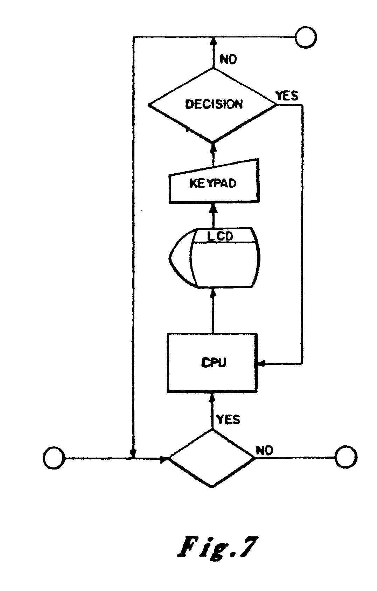

In Figure 6 are shown logical diagrams illustrating, in symbolic logic, the steps in the operation of the apparatus according to the invention, and Figure 7 illustrates a detail of one of the subroutines. The diamond-shaped boxes represent a decision, the trapezoidal boxes represent a keypad condition, the barrel-shaped boxes represent output displays, and the rectangular boxes represent CPU processing. The process will be better understood by considering the following example in the decision tree of Figure 6.

With reference again to Figure 5, the invention disclosed has the following Key Matrix. The "on" Key prompts the initial decision on which the rescuer must take action; Is the victim Adult, Baby or Child, (A,B or C)? This response, when keyed, leads the rescuer through a series of emergency techniques and then asks for a decision on the rescuer's diagnosis of the victim's condition and the rescue procedure required. The choices are:

1. One-Rescuer CPR
2. Two-Rescuer CPR
3. Mouth-to-Mouth Breathing
4. Choking - Victim Conscious
5. Choking - Victim Not Conscious

Once the rescuer decides on the "Age Logic Path," (A, B or C), the program stays with this path until the invention is restarted. If the condition of the victim changes or another rescuer joins the first rescuer, the rescuer can instantly change the logic path with keys 1, 2, 3, 4 and 5 in accordance with the new requirement. The logic for the preferred embodiment also includes provision for error messages and a pause key.

The operation of the apparatus will now be discussed with reference again to Figure 1. When a person trained in CPR is confronted with an unconscious or choking victim, he or she immediately checks vital signs and calls for help. The rescuer then locates the apparatus of the invention, which should be found in numerous places, much like a fire extinguisher. The invention is light and portable because it uses battery power, integrated circuits and a small case. The rescuer turns on the apparatus and listens for the prompts that will help diagnose the victim and initiate the CPR procedure.

After diagnosing the condition and age of the victim, the rescuer selects one of several logic paths through the input module 3. The choice might be such classifications as: Single-rescuer/multi-rescuer; Infant/child/adult; Choking/not choking; for example. The rescuer would then hear voice commands as output from the I.C. logic and perform the tasks as directed. The number of CPR repetitions would be accurate because the CPU logic would do the counting. The timing would be perfect for the same reason. Prompts would be heard at the proper time for diagnostic checks of breathing, pulse and other conditions.

If the condition of the victim changes, e.g. from "no pulse" to "pulse OK", the rescuer could key in this new information which would allow the central processing unit to search rapidly and select a new logic path. Should a second rescuer come upon the scene to help, again the logic path can be quickly changed with a keystroke.

The invention shows no fatigue, since an electronic chip has no moving parts. It performs calmly and faithfully until it is intentionally stopped or until the batteries wear down. It outlasts the stamina of most rescuers. The power consumption of these LSI chips is very low when operating. The built-in power standby modes may draw negligible power when not in use. For this reason simple AA size batters may power the apparatus for extremely long periods of time. The low power requirements of the LCD as compared with other display technology will also be beneficial.

The apparatus has the following further advantages:-

1. It prompts with audio sounds or voice to allow the rescuer free use of his hands, eyes, voice, mouth, and body.
2. It is independent of public utilities, having no reliance on telephone lines, A.C. electric outlets, or radio and cable circuits.
3. It employs conventional technology so that the apparatus is relatively inexpensive to manufacture, maintain, and use. This low cost helps to ensure that the apparatus will be just as available and prominent in public buildings as fire extinguishers and smoke detectors.

4. It is simple to understand and easy for one rescuer to operate.

5. It offers the user either keypad, menu, or punch-button input to initiate one of several CPR procedures published in the Red Cross CPR Module.

6. It gives the rescuer accurate timing for each CPR manoeuvre.

7. It gives the rescuer a proper count of repetitions in breathing and thrust routines.

8. It offers the rescuer calm, accurate instructions which will build confidence and lend encouragement to counteract the stress of emergencies.

9. It provides for easy logic changes should the instructions provided by, for example, the Red Cross CPR Module change in the future.

10. It makes provision for access ports to allow diagnostic enhancements in the future, such as direct pulse reading devices and output ports for modems.

11. It enables the rescuer to change the CPR prompts quickly should the condition of the victim change or should another rescuer appear on the scene to help.

12. It allows for international availability and use, being capable of supporting different languages.

13. In addition to its use in the field, the invention may be of special value in the schooling and recertification of rescuers, especially in that while it demonstrates the necessary rescue procedures, it offers perfect timing for the trainee. Instruction varies with regard to accurate timing for most CPR courses.

## Claims

1. Cardiopulmonary resuscitation prompting apparatus which includes

   keying means (A-C,3) for entering information signals representative at least of characteristics of a victim relevant to proper performance of cardiopulmonary resuscitation techniques; and

   means (21,24) responsive to the keyed information signals for providing output signals representative of proper steps to be taken in resuscitating a victim; characterised by

   electroacoustical transducing means (22,23,25,26) responsive to the output signals for providing audible intelligible speech sound signals representative of the proper resuscitation steps to be taken, and keying means (4, 5 in Fig. 5) for identifying whether the victim is conscious or unconscious.

2. Apparatus in accordance with claim 1, characterised in that the means responsive to the keyed information signals includes electronic speech synthesizing apparatus (14,15,22,23) for providing voice signals identifying the cardiopulmonary resusciation steps to be taken.

3. Apparatus in accordance with claim 2, characterised by circuit timing means (7,96) for providing voice prompts at the exact time a particular cardiopulmary resuscitation procedure step is to be taken.

4. Apparatus in accordance with any preceding claim, supported by a portable case, characterised in that the case also carries a source of electrical energy for powering the apparatus; and in that the keying means comprises a keyboard on a front panel of the case and includes means for turning the apparatus on, means (A,B,C) for identifying the nature of a victim, and means (1,2) for identifying the number of rescuers.

5. Apparatus in accordance with claim 4, characterised in that the case also carries an LCD display for furnishing visual information.

## Revendications

1. Appareil donnant des instructions pour la réanimation cardio-pulmonaire, qui comprend

   un moyen d'entrée par clavier (A à C, 3) pour entrer des signaux d'information représentatifs d'au moins les caractéristiques d'une victime relevant d'une exécution convenable des techniques de réanimation cardio-pulmonaire; et

   un moyen (21, 24) sensible aux signaux d'information entrés par clavier pour fournir des signaux de sortie représentatifs des étapes convenables devant être prises dans la réanimation d'une victime; caractérisé par un moyen

   transducteur électro-acoustique (22, 23, 25, 26), sensible aux signaux de sortie pour fournir des signaux de paroles intelligibles, audibles et représentatifs des étapes de réanimation convenables devant être prises.

2. Appareil selon la revendication 1, caractérisé en ce que le moyen sensible aux signaux d'information entrés au clavier comprend un appareil de synthése de paroles électronique (14, 15, 22, 23) pour fournir des signaux vocaux indentifiant les étapes de réanimation cardio-pulmonaires devant être effectuées.

3. Appareil selon la revendication 2, caractérisé par un moyen de cadencement de circuit (7, 96) pour fournir des instructions vocales au moment exact où une étape de procédure de réanimation cardio-pulmonaire doit être effectuée.

4. Appareil selon l'une quelconque des revendications précédentes, porté dans un coffret portable, caractérisé en ce que le coffret comporte également une source d'énergie électrique pour alimenter l'appareil; et en ce que le moyen d'entrée au clavier comprend un clavier sur une face avant du coffret et comprend un moyen pour mettre en marche l'appareil, un moyen (A, B, C) pour identifier la nature d'une victime, un moyen (1, 2) pour identifier le nombre de secouristes, et un moyen (4, 5) pour identifier si la victime est consciente ou inconsciente.

5. Appareil selon la revendication 4, caractérisé en ce que le coffret comporte également un affichage à LCD (24) pour fournir des informations visuelles.

**Patentansprüche**

1. Gerät zur Erteilung von Anweisungen für die kardio-pulmonale Wiederbelebung, enthaltend Eintastmittel zur Eingabe von Datensignalen, die zumindest charakteristische Merkmale eines Opfers repräsentieren, die für eine richtige Ausführung von kardio-pulmonalen Wiederbelebungsmethoden von Bedeutung sind, und auf die eingetasteten Daten ansprechende Mittel zur Lieferung von Ausgangssignalen, die die für die Wiederbelebung eines Opfers zu unternehmenden richtigen Schritte repräsentieren, gekennzeichnet durch auf die Ausgangssignale ansprechende elektroakustische Wandlereinrichtungen (22,23,25,26) zur Lieferung von akustischen verständlichen Sprach-Lautsignalen betreffend die zu unternehmenden richtigen Wiederbelebungsschritte und durch Eintastmittel (4,5 in Fig. 5) zum Identifizieren, ob das Opfer bei Bewußtsein oder bewußtlos ist.

2. Gerät nach Anspruch 1, dadurch gekennzeichnet, daß die auf die eingetasteten Datensignale ansprechenden Mittel einen elektronischen Sprachsynthesator (14,15,22,23) zur Lieferung von die zur kardio-pulmonalen Wiederbelebung zu unternehmenden Schritte kennzeichnenden Sprachsignalen enthalten.

3. Gerät nach Anspruch 2, gekennzeichnet durch Synchronisiermittel (7,96) zur Lieferung von gesprochenen Anweisungen zum richtigen Zeitpunkt, zu dem ein bestimmter Verfahrensschritt der kardio-pulmonalen Wiederbelebung auszuführen ist.

4. Gerät nach einem der vorhergehenden Ansprüche, das von einem tragbaren Gehäuse getragen wird, dadurch gekennzeichnet, daß das Gehäuse auch eine Stromquelle für die Stromversorgung des Geräts trägt, und daß die Eintastmittel eine Tastatur an einer Frontplatte des Gehäuses umfassen und Mittel zum Einschalten des Geräts, Mittel (A,B,C) zur Identifizierung der Natur eines Opfers und Mittel zur Identifizierung der Anzahl der Helfer enthalten.

5. Gerät nach Anspruch 4, dadurch gekennzeichnet, daß das Gehäuse auch eine LCD-Anzeige zur Lieferung sichtbarer Information trägt.

Fig. 1

EXTERNAL ROM 23

EXTERNAL ROM 23

27 POWER

OR V°

23 EXTERNAL ROM 122 K BITS 100 SECONDS SYNTHESIZED SPEECH

SPEAKER, 26

32Ω

VSS (M)

220 pf

400 KH₂

1KΩ

200pf

22 CMOS SINGLE CHIP LSI THAT ENABLE 20 SECONDS OF SPEECH SYNTHESIS USING PARCOR METHOD

.022 uf

240Ω

1uf

1KΩ

25 AUDIO AMPLIFIER

$V_{DD}^+$ (M)

120pf

32768

21 CMOS 4-BIT MICROCOMPUTER

KEY MATRIX KEYBOARD

COMPENSATOR, 28

24 LIQUID CRYSTAL DISPLAY

**Fig. 2**

Fig.3

10

Fig.4

*Fig.5*

Fig.6

EP 0 183 462 B1

*Fig.7*